(19) Europäisches Patentamt
European Patent Office
Office européen des brevets

(11) Publication number: **0 614 662 A1**

# (12) EUROPEAN PATENT APPLICATION

(21) Application number: **94103055.3**

(51) Int. Cl.[5]: **A61K 31/20**

(22) Date of filing: **01.03.94**

(30) Priority: **11.03.93 JP 76388/93**
**08.11.93 JP 300806/93**

(43) Date of publication of application:
**14.09.94 Bulletin 94/37**

(84) Designated Contracting States:
**CH DE FR GB IT LI NL**

(71) Applicant: **Eisai Co., Ltd.**
**6-10, Koishikawa 4-chome**
**Bunkyo-ku**
**Tokyo (JP)**

(72) Inventor: **Tsurumi, Hisashi**
**23-21, Noritakenaka 1-chome**
**Gifu-shi, Gifu (JP)**
Inventor: **Takahashi, Takeshi**
**1-20, Fukuzumicho**
**Gifu-shi, Gifu (JP)**
Inventor: **Moriwaki, Hisataka**
**11, Kaguracho**
**Gifu-shi, Gifu (JP)**
Inventor: **Muto, Yasutoshi**
**3459-91, Nagara**
**Gifu-shi, Gifu (JP)**
Inventor: **Asano, Shigetaka**
**20-25, Ohmiya 1-chome**
**Suginami-ku, Tokyo (JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

## (54) Use of (2E,4E,6E,10E)-3,7,11,15-tetramethyl-2,4,6,10,14-hexadecapentaenoic acid for the treatment of leukaemia.

(57) To provide a remedy or ameliorant, based on the effect of inducing cell differentiation, for diseases such as hematopoietic organ tumors and solid tumors for which no clinically highly useful drug has been known hitherto. The fundamental idea of the conventional pharmacotherapy for cancer resides in completely exterminating tumor cells having an unusually high capability of multiplication. However these treatments also had toxicities against normal cells and thus grave side effects were unavoidable. Further, only limited therapeutic effects could be achieved. On the other hand, although all-transretinoic acid had a remarkable effect on acute promyelocytic leukemia, there was reported that this compound also had grave side effects. Further, there was another problem that the diseases tended to recur after it was healed. In contrast, (2E,4E,6E,10E)-3,7,11,15-tetramethyl-2,4,6,10,14-hexadecapentaenoic acid has an unexpected effect of inducing cell differentiation and a high safety. Being different in structure from all-trans-retinoic acid, this compound has a high possibility of preventing the recurrence of the disease. Therefore, it is expected as a clinically useful remedy or ameliorant for hematopoietic organ tumors, in particular, acute promyelocytic leukemia and myeloid dysplasia syndrome.

EP 0 614 662 A1

This invention relates to the use of (2E, 4E, 6E, 10E)-3,7,11,15-tetramethyl-2,4,6,10,14-hexadecapentaenoic acid or a pharmacologically acceptable salt thereof in the preparation of a remedy or ameliorant for hematopoietic organ tumors on the basis of the effect of inducing cell differentiation (hereinafter referred to as inducing differentiation).

[Background of the Invention]

Cancers (tumors) are classified in various ways depending on, for example, manifestation sites, pathological images and conditions. Leukemia, which is a representative disease of hematopoietic organ tumors and means tumors in blood cells (leukocytes), is marked by the multiplication of various undifferentiated blast leukocytes. Further, leukemia is subdivided into acute leukemia and chronic leukemia wherein the tumor cells being under multiplication are immature blasts and matured cells, respectively. Thus there are observed various clinical symptoms of leukemia, though most of them can be roughly classified into symptoms due to the suppression of normal hemopoiesis and those due to the infiltration and/or compression of other organs. More particularly, a fall in normal blood cells is expressed in anemia caused by erythropenia, infectious diseases and flush caused by granulopenia and a tendency toward hemorrhage caused by thrombocytopenia, while the suppression of normal hemopoiesis induces bone marrow insufficiency. It is well known in general that leukemia is a disease with anxious prognosis. Thus there have been examined various drugs and therapeutic methods therefor.

Among acute leukemias acute promyelocytic leukemia (hereinafter referred to simply as APL) is a disease where the differentiation of tumor cells stops in the step of promyelocytes and a number of coarse granules and promyelocytes haying a highly atypical nucleus undergo multiplication. In the hematological FAB classification, APL falls within the category of type M3 and is characterized by being accompanied by chromosomal aberration t (15 : 17). From a clinical viewpoint, disseminated intravascular coagulation syndrome (DIC) caused by tissue thromboplastin originating in coarse granules frequently takes place in the case of APL. Furthermore, APL is accompanied by intense fibrinolysis sthenia due to fibrinolytic activators such as plasminogen activator and elastase liberated from leukemic cells and severe hemorrhage is thus induced. Namely, APL is the severest or gravest disease among acute leukemias.

On the other hand, there is another type of acute leukemia called myeloid dysplasia syndrome (hereinafter referred to simply as MDS) where blasts increase so as to give a preleukemic blood image but do not exceed the level of 30% in the bone marrow. In the case of MDS, although no clinical symptom appears immediately, blasts increase with the lapse of time. Thus there is a high possibility that MDS would progress into APL.

The fundamental idea of the conventional pharmacotherapy for the above-mentioned pathosis resides in achieving a therapeutic effect by completely exterminating the leukemic cells, i.e., the tumor cells. Accordingly attempts have been made to cope with tumors having an unusually high ability to multiply through the development of a drug having an elevated cytotoxicity and thus being capable of exerting a more potent cytotoxic effect or the administration of the existing drugs together with other concomitant drugs, at a high concentration or in a large dose so as to achieve improved therapeutic results. However, these drugs and treatments not only exert the effects specifically on tumor cells but also have toxicities against normal cells and grave side effects such as heart and cardiac muscle disorders, suppression of bone marrow function, vomiturition, vomiting, neuropathy and depilation are thus induced. As a result, only limited therapeutic effects could have been achieved heretofore.

On the other hand, it has been known that various differentiation inducers, which are superior in safety to the conventional anticancer agents, induce the differentiation of tumor cells into mature cells in vitro and thus the induction of differentiation is highly expected as a useful therapeutic method. However it is to be regretted that none of the conventional differentiation inducers has proved any clinical usefulness. However, since Huang et al. reported clinical data showing that the use of all-trans-retinoic acid (hereinafter referred to simply as ATRA) represented by the following structural formula:

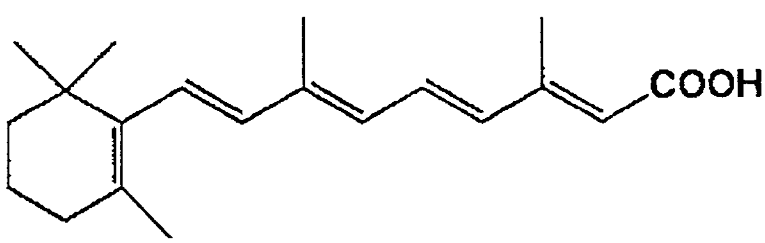

resulted in an almost complete (about 100%) healing of APL patients in 1988 [see Blood, 72, 567 - 572 (1988)], this effect has been followed up and reconfirmed in many countries in the world. Thus there is the ever-increasing expectancy of the differentiation-inducing method being effective not only in the treatment of hematopoietic organ tumors but also in the treatment of cancers over a wide range including solid tumors.

As described above, there has been reported that ATRA is clinically effective on APL by a number of workers including Huang et al. [see Blood, 72, 567 - 572 (1988)], Castaigne at al. [see Blood, 76, 1704 - 1709 (1990)] and Warrell [see New Engl. J. Med., 324, 1385 - 1393 (1991)].

Further, Olsson et al. have reported that 1$\alpha$,25-dihydroxycholecalciferol, which is a physiologically active metabolite of vitamin $D_3$ and hereinafter referred to simply as active $VD_3$), has the effect of inducing differentiation on a cultured human lymphoma cell line (U937) [see Cancer Res., 43 (12Pt1), 5862 - 5867 (1983)]. Subsequently, attempts have been actively made to develop active $VD_3$ derivatives. For example, Japanese Patent Publication-A No. 33165/1986 has disclosed that a 24-alkyl-dehydrovitamin $D_3$ derivative has an antitumor effect, while Japanese Patent Laid-Open No. 140560/1986 has disclosed that 20-oxa-21-norvitamin $D_3$ derivative has the effect of inducing differentiation.

Zhang et al. have reported that bufalin has the effect of inducing differentiation on cultured human leukemic cell lines HL-60, U937 and ML1 [see Biochem. Biophys. Res. Commun., 178 (2), 686 - 693 (1991) and Cancer Res., 52 (17), 4634 - 4641 (1992)].

In addition to the above-mentioned compounds, there have been reported cytosine arabinose (Ara-C) [see Baccarani et al., Br. J. Haematol 42, 485 - 487 (1979)], aclacinomycin A [see Morin et al., Cancer Res., 44, 2807 - 2812 (1984)] and interferon $\alpha$ [see Moriya et al., Jap. J. Clin. Hepatol. 32, 170 - 172 (1991)] as the compounds each having the effect of inducing differentiation.

By using a cultured cell line of mouse myelocytic leukemia, Ishikura et al. have reported that geranylfarnesol (3,7,11,15,19-pentamethyl-2,6,10,14,18-eicosapentaen-1-ol) has the effect of inducing differentiation [see Leukemia Res., 8(5), 843 - 852 (1984)].

ATRA and its derivatives have been applied to the treatment of skin cancer and psoriasis which is a intractable skin keratosis. However, these compounds have side effects leading to even death [see Annals of Internal Medicine, 117, 292 - 296 (1992)]. Further, it is widely known that the prolonged administration of these compounds frequently induce hypervitaminosis A including leukocytosis, dryness in skin and lips, dermatitis, gastrointestinal disorders, ostealgia, hyperlipemia and hepatopathy such as hepatic auxesis due to the extremely high solubility in fat. In such a case, ATRA or its derivative will remain in the liver or other organs for a long period of time even after ceasing the administration. Namely, there is a serious disadvantage that once these side effects occur, they last for a prolonged period of time. As described above, ATRA is effective in treating APL. However, there is another problem that the APL tends to recur even when ATRA is repeatedly administered after healing.

Vitamin $D_3$ derivatives have been used in the treatment of, for example, osteoporosis. These compounds accelerate the absorption of calcium in the intestine and the reabsorption of calcium in the kidneys. Therefore, it is known that the administration of these compounds in an excessive dose induces hypercalcemia and thus result in nephropathy and disorders in the digestive system due to calcinosis. Accordingly, it is necessary to regularly examine the serum calcium level during the period of the administration of these compounds, which makes them hardly usable in the clinical medicine. In addition, although the vitamin $D_3$ derivatives have the effect of inducing differentiation in a cultured cell line HL60 originating in human acute promyelocytic leukemia, no such an efficacy is observed in any models of other types.

Since bufalin has not been used clinically so far, the safety thereof remains completely unknown. It is therefore impossible to estimate the usefulness of this compound in man.

On the other hand, neither cytosine arabinoside nor aclacinomycin A has been accepted as a drug in Japan because of the problem of safety. In addition, the antitumor effect of interferon $\alpha$ falls short of expectations.

The data on the evaluation of the differentiation induction effect of geranylfarnesol are ones obtained by using a cultured mouse leukemic cell line and no data in a cultured human laukemic cell line has been reported hitherto. When, therefore, a difference in the sensitivity of cells to drug from species to species is taken into consideration, the efficacy of this compound in man is completely unknown.

Accordingly no existing drug has an excellent efficacy on hematopoietic organ tumors, in particular, APL and MDS together with a high safety. Thus there has been urgently required to develop a medicine which is highly useful for cancers over a wide range in the clinical medicine.

U.S. patents 4 917 829 and 4 988 732 show the compound of the invention and a first pharmacological use thereof.

[Description of the Invention]

[Problems to Be Solved by the Invention]

The (2E,4E,6E,10E)-3,7,11,15-tetramethyl-2,4,6,10,14-hexadecapentaenoic acid (I) [hereinafter referred to simply as the compound (I)] according to the present invention is represented by the following chemical formula:

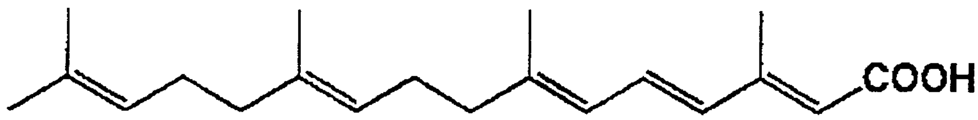

(I)

It has been disclosed in, for example, US-A 4 988 732 and Japanese Patent Publication-A No. 181012/1982 as a compound having an anticancer effect and the effect of ameliorating dermatological diseases accompanied by keratosis. It has been confirmed that this compound (I) exerts an antitumor effect in mouse papilloma. In a toxicity test with the use of mice, an ATRA-administration group suffered from hypervitaminosis A including depilation and reduction in body weight, while a compound (I)-administration group did not suffer from such a symptom. Thus it has been proved that the compound (I) has a very low toxicity. The present inventors have paid their attention to the fact that the compound (I) has both of various physiological activities and a low toxicity and have examined its efficacy on other diseases for a long period of time. As a result, they have unexpectedly found out that this compound (I) has a differentiation-inducing effect and is therefore available in achieving the above-mentioned object as a remedy or ameliorant for hematopoietic organ tumors. Thus the present invention has been completed.

Accordingly, it is an object of the present invention to provide a remedy or ameliorant for hematopoietic organ tumors which has a differentiation-inducing effect and a high clinical usefulness. More particularly, the present invention relates to a differentiation inducer, still particularly a remedy or ameliorant for hematopoietic organ tumors, which contains the compound (I) represented by the Chemical formula 3 or a pharmacologically acceptable salt thereof as an active ingredient. As particular examples of the hematopoietic organ tumors, acute leukemia including APL and MDS, chronic leukemia, malignant lymphoma, multiple myeloma and macroglobulinemia may be cited. The cell differentiation inducer of the present invention is particularly effective in treating APL and MDS, though it is needless in say that the diseases to be treated thereby are not restricted to them.

The invention also provides a method for inducing cell differentiation of a mammal subject by administering a pharmacologically effective amount of (2E, 4E, 6E, 10E)-3,7,11,15-tetramethyl-2,4,6,10,14-hexadecapentaenoic acid or a pharmacologically acceptable salt thereof to said subject. In the method of the invention, it is preferable that the mammal subject is a human subject; hematopoietic organ tumors are therapeutically treated; and acute promyelocytic leukemia or myeloid dysplasia syndrome are therapeutically treated or ameliorated.

The invention provide use of (2E, 4E, 6E, 10E)-3,7,11,15-tetra-methyl-2,4,6,10,14-hexadecapentaenoic acid or a pharmacologically acceptable salt for manufacturing a medicine thereby to induce cell differentiation, that is, a cell differentiation inducer.

The invention then provides a cell differentiation inducer comprising (2E, 4E, 6E, 10E)-3,7,11,15-tetramethyl-2,4,6,10,14-hexadecapentaenoic acid or a pharmacologically acceptable salt and a pharmacological composition to induce cell differentiation, comprising a pharmacologically effective amount of (2E, 4E, 6E, 10E)-3,7,11,15-tetramethyl-2,4,6,10,14-hexadecapentaenoic acid or a pharmacologically acceptable salt thereof and a pharmacologically acceptable carrier.

The compound to use in the invention has the above shown formula (I), being one of acyclic polyisoprenoid compounds.

It is believed that the mechanism of the remarkable effect of ATRA on APL is based on a phenomenon that a reciprocal translocation occurs between the chromosomes 15 and 17 in an APL cell and the center gene PML located on the chromosome 15 and the retinoic acid receptor α (RAR-α) located on the chromosome 17 form together a fused gene PML/RAR-α as the reciprocal translocation proceeds, thus suppressing the differentiation of the APL cell. Accordingly, it is expected that the detection of this fused

gene PML/RAR-α will enable a more appropriate pharmacotherapy for APL in future. However there has been no substance proved to have an efficacy on PML/RAR-α positive cells except ATRA. Thus the compound (I) according to the present invention and its pharmacologically acceptable salts are compounds the efficacies of which on PML/RAR-α positive cells have been proved for the first time except ATRA.

As described above, there is a problem that APL tends to recur even when it has been healed with the use of ATRA which has been continuously administered thereafter. This is seemingly because the prolonged administration of ATRA results in hepatic drugmetabolizing enzyme induction and thus the metabolism of ATRA per se is accelerated, which makes it impossible to maintain the concentration of ATRA in the blood at an appropriate level [see Cancer Research, 52, 2138 - 2142 (1992) and Blood, 79 (2), 299 - 303 (1992)]. Regarding this point, it is expected that the compound (I) of the present invention, which is an acyclic isoprenoid and entirely different in structure from ATRA which is a cyclic isoprenoid, will not cause hepatic drug-metabolizing enzyme induction. Thus it is expected that the compound (I) or its pharmacologically acceptable salt will be surely effective in the clinical medicine.

The compound (I) according to the present invention can be prepared in accordance with a method described in Japanese Patent Publication No. 32058/1988 by using, for example, farnesylacetone as a starting material. The pharmaceutically acceptable salt to be used in the present invention is not restricted, so long as it is a salt formed with the compound (I). Particular examples thereof include alkali metal addition salts such as sodium, potassium and lithium salts, alkaline earth metal addition salts such as calcium and magnesium salts, ammonium salt, amine addition salts such as methylamine, ethylamine, diethylamine, triethylamine and ethanolamine salts, and amino acid addition salts.

Next the results of an acute toxicity test on the compound of the present invention will be given.

[Acute Toxicity Test]

(Method)

By using 5 female and 5 male SD rats and ICR mice aged 7 to 8 weeks, the toxicities of the compound of the present invention were tested through oral, intramuscular, subcutaneous and intraperitoneal administrations each in a single dose (medium: 5% gum arabic).

(Result)

The following Table 1 summarizes the $LD_{50}$ (mg/kg) data.

Table 1

| Acute toxicity (mg/kg) of compound (I) [(2E,4E,6E,-10E)-3,7,11,15-tetramethyl-2,4,6,10,14-hexadecapentaenoic acid] | | | | | |
|---|---|---|---|---|---|
| Animal | Sex | Oral | Intramuscular | Subcutaneous | Intraperitoneal |
| Mouse (ICR) | ♂ | >6,250 | 2.000 | >4,000 | 970 |
| | ♀ | >6,250 | >2,000 | >4.000 | 1.100 |
| Rat (SD) | ♂ | 2,400 | 830 | 2,500 | 731 |
| | ♀ | 2,850 | 1.120 | 2,800 | 680 |

As Table 1 clearly shows, the compound of the present invention has a high $LD_{50}$ value and, therefore, an extremely high safety.

As examples of the dosage form, oral preparations such as powders, fine subtilaes, granules, tablets, coated tablets and capsules, preparations for external use such as ointments and cataplasms and injections may be cited. These preparations can be produced by the conventional methods with the use of pharmaceutical carriers commonly employed in the art.

More specifically, oral preparations are formulated by blending the compound (I) or a pharmacologically acceptable salt thereof with fillers and, if required, binders, disintegrators, lubricants, coloring agents and corrigents and then formulating into powders, fine subtilaes, granules, tablets, coated tablets or capsules in the conventional manner.

Examples of the fillers include lactose, corn starch, sucrose, glucose mannitol, sorbitol, crystalline cellulose and silicon dioxide. Examples of the binders include polyvinyl alcohol, polyvinyl ether, methylcellulose, ethylcellulose, gum arabic, tragacanth, gelatin, shellac, hydroxypropylmethylcellulose, polyvinylpyrrolidone, polypropylene glycol/polyoxyethylene block polymer and meglumine. Examples of the disintegrators include starch, agar, gelatin powder, crystalline cellulose, calcium carbonate, sodium hydrogencarbonate, calcium citrate, dextrin, pectin and carboxymethylcellulose calcium. Examples of the lubricants include magnesium stearate, talc, polyethylene glycol, silica and hardened vegetable oils. As the coloring agents, those which have been approved as pharmaceutical additives are usable. Examples of the corrigents include cocoa powder, menthol, aromatic powder, peppermint oil, borneol and powdered cinnamon bark. Needless to say, these tablets and granules may be optionally coated with sugar or other substances, if necessary.

To formulate injections, the compound (I) or its parmacologically acceptable salt is blended with pH regulators, solvents, tonicity agents and, if required, solubilizing agents, stabilizers, etc., and then formulated into injections in the conventional manner.

Methods for formulating preparations for external use are not restricted. Thus they can be produced by the conventional methods. Namely, various materials commonly employed in drugs, quasi drugs and cosmetics are usable as a base material.

Particular examples of the base material include animal and vegetable oils, mineral oils, ester oils, waxes, higher alcohols, fatty acids, silicone oil, surfactants, phospholipids, alcohols, polyhydric alcohols, water-soluble polymers, clay minerals and purified water. If necessary, pH regulators, antioxidants, chelating agents, antiseptics, fungicides, coloring agents and perfumes may be added thereto. It is to be understood that the base material to be used in the preparations for external use according to the present invention are not restricted to those cited above. Furthermore, these preparations may contain other ingredients such as differentiation inducers, blood circulation accelerators, bactericides, antiphotogistics, cell potentiators, vitamins, amino acids, humectants and keratolytic agents, if necessary. These base materials are added in such an amount as to adjust the content thereof to a level commonly sat in the formulation of preparations for external use.

In the present invention, the clinical dose of the compound (I) or its pharmacologically acceptable salt is not particularly restricted but varies depending on the condition, severity, age and complication. Further, it varies depending on the type of the salt and the administration route. Usually, the dose ranges from 100 to 1200 mg, preferably from 300 to 1000 mg and still preferably from 500 to 700 mg, per day for an adult in the case of oral, intravenous or percutaneous administration.

To illustrate the usefulness of the compound of the present invention as a differentiation inducer, a test example on the effects of the compound of the present invention on leukemic cells of APL patients and various cultured human leukemic cell lites will be given. The backgrounds of the patients providing the bloods employed in the test are as follows.

Table 2

| Patient | Age | Sex | FAB type | Bone marrow | | Chromosome | Fused gene PML/RAR-α |
|---|---|---|---|---|---|---|---|
| | | | | (NCC)* $\times 10^4$ | blast content (%) | | |
| 1 | 46 | M | M3/recurrence | 35.2 | 45 | 46XY, t(15:17) | (+) |
| 2 | 49 | M | M3/initiation | 80.0 | 95 | 46XY | (+) |
| 3 | 48 | F | M3/recurrence | 13.9 | 94 | 46XX, t(15:17) | (+) |

NCC*; nucleated cell counts

[Brief Description of the Drawings]

Fig. 1 is a graph showing the relationship between the concentration of the compound (I) or ATRA and the cell count of a subcultured human APL cell strain HL-60 (expressed in average ± standard deviation).

Fig. 2 is a graph showing the relationship between the concentration of the compound (I) or ATRA and the viable cell count of HL-60.

(Method)

In the case of HL-60 which was a subcultured human APL cell line, the initial cell count was adjusted to $5 \times 10^4$ cells/ml and incubation was effected in an IMDM medium (mfd. by Gibco) containing 10% of fetal calf serum for 5 days under the addition of compound (I) or ATRA ($10^{-4}$ to $10^{-8}$ mol/1). After the completion of the incubation, the total cell count was calculated with a Burker-Turk calculation board and the viable cell count was calculated by regarding the cells stained with Trypan Blue as the dead ones. Thus $ID_{50}$ and $LD_{50}$ were determined. Further, a cytospin slide was prepared and the morphology of the cells was observed by the May-Giemsa staining method.

The cells samples from APL patients were adjusted to an initial cell count of $1 \times 10^6$ cells/ml and incubated in the above-mentioned IMDM medium containing 10% of fetal calf serum for 5 days under the addition of the compound (I) or ATRA ($10^{-6}$ to $10^{-8}$ mol/1), followed by the same evaluation as the one effected in the case of HL-60. Further, the ratio of mature cells to all the cells was determined in accordance with the following formula and taken as an indication for the comparison of the ability to induce differentiation:

$$\text{ratio of mature cells (\%)} = \frac{[\text{myelocyte count} + \text{metamyelocyte count} + \text{rod nuclear cell count} + \text{segmented cell count}]}{\text{total cell count}} \times 100$$

(Results)

(1) Multiplication-inhibiting effect on HL-60

Fig. 1 shows the relationship between the concentration of the compound (I) or ATRA and the HL-60 cell count, while Fig. 2 shows the relationship between the above concentration and the viable cell count.

As Fig. 1 clearly shows, the compared (I) and ATRA each inhibited the multiplication of the HL-60 cells with an increase in the concentration and an obvious effect of suppressing cell differentiation was observed at a concentration of $7.5 \times 10^{-6}$ mol/l or above. The $ID_{50}$ of the compound (I) was $1.0 \times 10^{-5}$ mol/l and that of ATRA was $1.4 \times 10^{-5}$ mol/l.

As Fig. 2 clearly shows, the compound (I) and ATRA each caused a decrease in the viable cell count at a concentration of $1.0 \times 10^{-5}$ mol/l or above. However, each of these compounds exhibited a low cytotoxicity at a concentration lower than the abovementioned level and the ratio of the dead cells stained with Trypan Blue was extremely low (about 10% or less). The $LD_{50}$ of the compound (I) determined from Fig. 2 was $3.8 \times 10^{-5}$ mol/l and that of ATRA was $2.5 \times 10^{-5}$ mol/l.

It has been thus clarified that both of the compound (I) and ATRA induce the differentiation of HL-60 cells. To clarify the characteristics of these compounds, the $LD_{50}/ID_{50}$ ratios, i.e., an indication of the margin of safety were determined. Table 3 shows the results.

Table 3

| Comparison of compound (I) and ATRA in the margin of safety ($LD/_{50}/ID_{50}$ ratio) | | | |
|---|---|---|---|
| | $ID_{50}$ (mol/l) | $LD_{50}$ (mol/l) | $LD_{50}/ID_{50}$ |
| compound (I) | $1.0 \times 10^{-5}$ | $3.8 \times 10^{-5}$ | 3.80 |
| ATRA | $1.4 \times 10^{-5}$ | $2.5 \times 10^{-5}$ | 1.79 |

As Table 3 clearly shows, the margin of safety expressed in the $LD_{50}/ID_{50}$ ratio of the compound (I) is about 2.1 times as high as that of ATRA. Therefore it is expected that the compound (I) is more useful than ATRA in the clinical medicine.

(2) Abilities of compound (I) and ATRA to induce differentiation

Table 4 shows the abilities of the compound (I) and ATRA to induce differentiation of HL-60 cells and Table 5 shows the abilities of these compounds to induce differentiation of cells of APL patients.

[Table 4]

Ability to induce differentiation of HL-60 cells

| | Blast (%) | Promyelocyte (%) | Myelocyle (%) | Metamyelocyte (%) | Rod nuclear cell (%) | Segmented cell (%) | differentiation ratio (%) |
|---|---|---|---|---|---|---|---|
| control | 2 | 91 | 7 | 0 | 0 | 0 | 7 |
| compound (I) | 0 | 8 | 42 | 20 | 17 | 13 | 92 |
| ATRA | 1 | 12 | 41 | 19 | 19 | 8 | 87 |

[Table 5]

Ability to induce differentiation of APL cells

| Patient | | Blast (%) | Promyelocyte (%) | Myelocyte (%) | Metamyelocyte (%) | Rod nuclear cell (%) | Segmented cell (%) | Differentiation ratio (%) |
|---|---|---|---|---|---|---|---|---|
| 1 | control | 4 | 90 | 6 | 0 | 0 | 0 | 6 |
| | compound (I) | 0 | 12 | 38 | 35 | 12 | 3 | 88 |
| | ATRA | 1 | 9 | 40 | 28 | 17 | 5 | 90 |
| 2 | control | 3 | 92 | 5 | 0 | 0 | 0 | 5 |
| | compound (I) | 2 | 14 | 48 | 32 | 2 | 2 | 84 |
| | ATRA | 2 | 14 | 45 | 30 | 8 | 1 | 84 |
| 3 | control | 5 | 88 | 4 | 1 | 2 | 0 | 7 |
| | compound (I) | 4 | 17 | 59 | 17 | 3 | 0 | 79 |
| | ATRA | 5 | 15 | 54 | 20 | 4 | 2 | 80 |

As Table 4 clearly shows, when the HL-60 cells were morphologically observed after the incubation, a sufficient effect of inducing differentiation was observed at a concentration of the compound (I) of $1.0 \times 10^{-6}$ mol/l or above or at a concentration of ATRA of $1.0 \times 10^{-7}$ mol/l or above.

On the other hand, in the examination with the use of the cells of APL patients, the compound (I) exhibited an effect of inducing differentiation similar to ATRA. Furthermore, the compound (I) was effective in all of the cases of cells having a fused gene PML-RAR-$\alpha$ on which it had been believed that only ATRA was effective. Accordingly, it may be thought that the examination on this gene in APL patients conducted as a routine test will enable the practice of more appropriate pharmacotherapy. When the abovementioned high margin of safety of the compound (I) is taken into consideration too, it is obvious that the compound (I) or its pharmacologically acceptable salt can be expected to be highly useful in the clinical medicine.

## Claims

1. The use of (2E, 4E, 6E, 10E)-3,7,11,15-tetramethyl-2,4,6,10,14-hexadecapentaenoic acid or a pharmacologically acceptable salt thereof in the manufacture of a cell differentiation inducer.

2. The use according to claim 1, wherein the cell differentiation inducer is effective against hematopoietic organ tumors.

3. The use according to claim 2, wherein the hematopoietic organ tumor is acute promyelocytic leukemia or myeloid dysplasia syndrome.

4. The use according to any of the preceding claims, characterized in that the cell differentiation inducer is effective in human beings.

Fig. 1

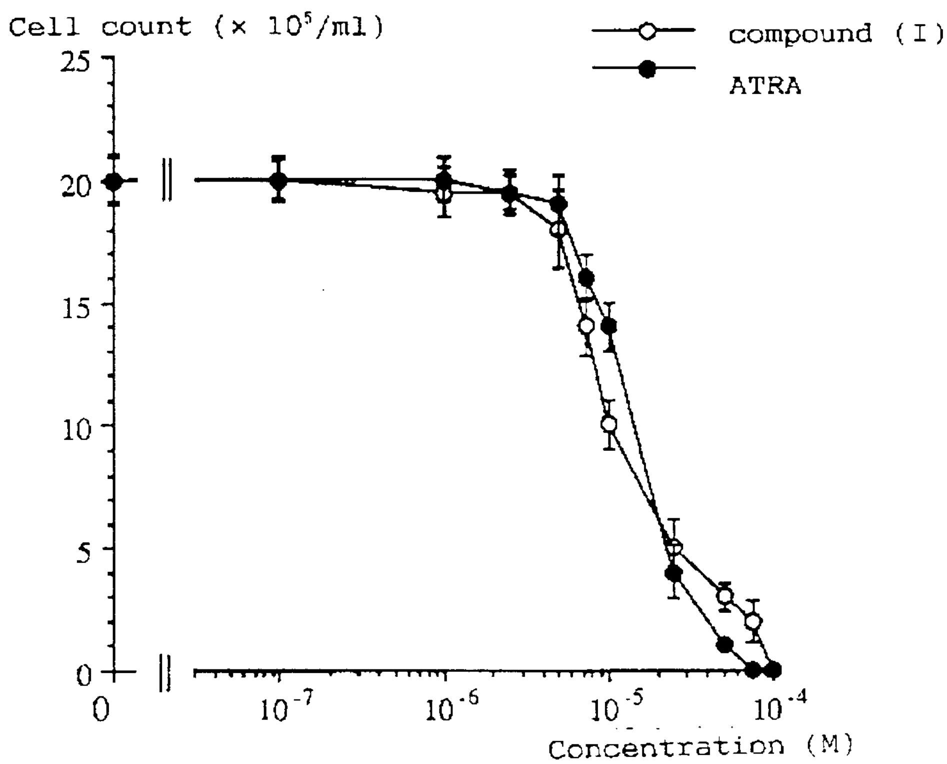

Cell count (× $10^5$/ml)
compound (I)
ATRA
25
20
15
10
5
0
0
$10^{-7}$
$10^{-6}$
$10^{-5}$
$10^{-4}$
Concentration (M)

Fig. 2

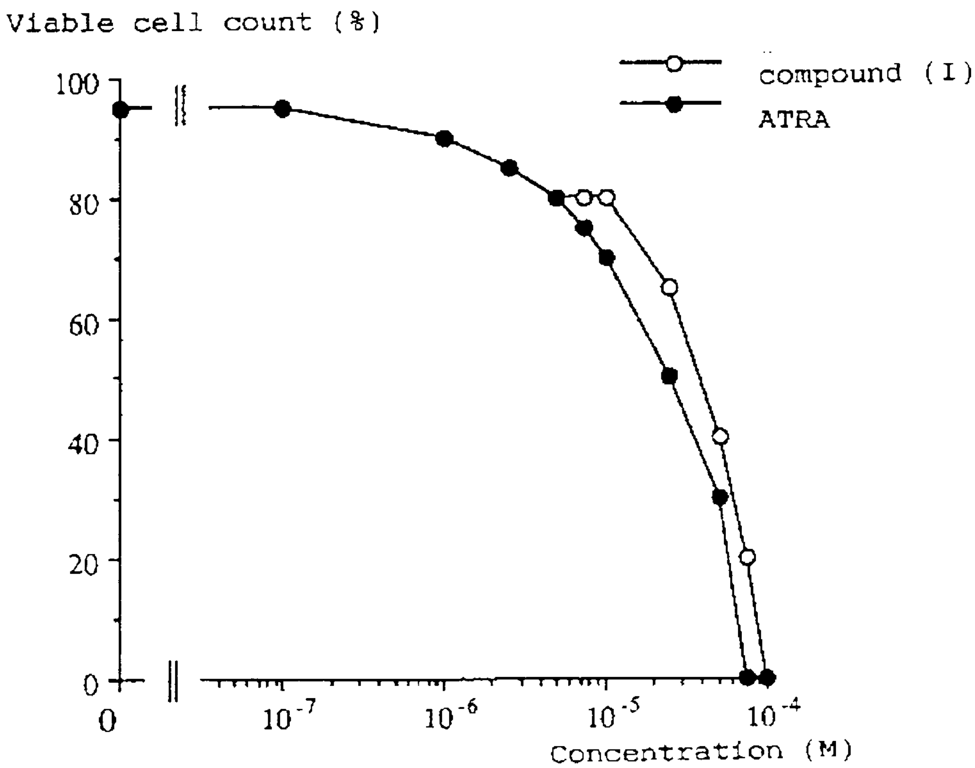
Viable cell count (%)
100
80
60
40
20
0
0
$10^{-7}$
$10^{-6}$
$10^{-5}$
$10^{-4}$
Concentration (M)
compound (I)
ATRA

European Patent Office

# EUROPEAN SEARCH REPORT

Application Number
EP 94 10 3055

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| P,X | INTERNATIONAL JOURNAL OF HEMATOLOGY vol. 59, no. 1 , December 1993 pages 9 - 15 TSURUMI, H. ET AL 'DIFFERENTIATION INDUCTION OF HUMAN PROMYELOCYTIC LEUKEMIA CELLS BY ACYCLIC RETINOID (POLYPRENOIC ACID)' * the whole document * --- | 1-4 | A61K31/20 |
| X | THE BULLETIN OF THE YAMAGUCHI MEDICAL SCHOOL vol. 33, no. 1-4 , December 1986 pages 1 - 7 SASAKI, K. ET AL 'INDUCTION TO GRANULOCYTIC DIFFERENTIATION OF HUMAN PROMYELOCYTIC LEUKEMIA CELLS (HL-60) BY POLYPRENOIC ACID (E5166)' * the whole document * --- | 1-4 | |
| D,A | LEUKEMIA RESEARCH vol. 8, no. 5 , 1984 pages 843 - 852 ISHIKURA, H. ET AL 'DIFFERENTIATION OF MOUSE LEUKEMIC M1 CELLS INDUCED BY POLYPRENOIDS' * the whole document * --- | 1-4 | TECHNICAL FIELDS SEARCHED (Int.Cl.5) A61K |
| A | GANN (JAPANESE JOURNAL OF CANCER RESEARCH) vol. 72, no. 6 , December 1981 pages 974 - 977 MUTO, Y. ET AL 'IN VITRO BINDING AFFINITY OF NOVEL SYNTHETIC POLYPRENOIDS (POLYPRENOIC ACIDS) TO CELLULAR RETINOID-BINDING PROTEINS' * the whole document * --- -/-- | 1-4 | |

The present search report has been drawn up for all claims

| Place of search | Date of completion of the search | Examiner |
|---|---|---|
| THE HAGUE | 26 April 1994 | Mair, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application Number
EP 94 10 3055

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | JAPANESE JOURNAL OF CANCER RESEARCH<br>vol. 81, no.12 , December 1990<br>pages 1281 - 1285<br>FUKUTOMI, Y. ET AL 'INHIBITORY EFFECTS OF ACYCLIC RETINOID (POLYPRENOIC ACID) AND ITS HYDROXY DERIVATIVE ON CELL GROWTH AND ON SECRETION OF ALPHA-FETOPROTEIN IN HUMAN HEPATOMA-DERIVED CELL LINE (PLC/PRF/5)'<br>* the whole document *<br>----- | 1-4 | |
| | | | TECHNICAL FIELDS SEARCHED (Int.Cl.5) |

The present search report has been drawn up for all claims

| Place of search | Date of completion of the search | Examiner |
|---|---|---|
| THE HAGUE | 26 April 1994 | Mair, J |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document